# EUROPEAN PATENT SPECIFICATION

(11) **EP 0 572 517 B1**
(45) Date of publication and mention of the grant of the patent: **08.07.1998**
(21) Application number: 92906766.8
(22) Date of filing: 14.02.1992
(51) Int. Cl.: C07C 2/00, C07C 29/50, C07C 31/04, C07C 67/39, C07C 303/24, C07C 303/26, C07C 303/28, C07C 305/06, C07C 391/00

(54) **CATALYTIC PROCESS FOR CONVERTING HYDROCARBONACEOUS FEEDS, PARTICULARLY LOWER ALKANES TO ESTERS, ALCOHOLS, AND OPTIONALLY TO HYDROCARBONS**
KATALYTISCHES VERFAHREN ZUM UMSETZEN VON KOHLENWASSERSTOFFSTRÖMEN, INSBESONDERE NIEDRIGE ALKANE, IN ESTER, ALKOHOLE UND GEGEBENENFALLS KOHLENWASSERSTOFFE
PROCEDE CATALYTIQUE DE CONVERSION DE CHARGES D'HYDROCARBURES, NOTAMMENT D'ALCANES INFERIEURS EN ESTERS, ALCOOLS, ET EVENTUELLEMENT EN HYDROCARBURES

(30) Priority: 15.02.1991 US 656910; 26.09.1991 US 766200; 27.11.1991 US 799446
(43) Date of publication of application: 08.12.1993
(73) Proprietor: CATALYTICA, INC., Mountain View, CA 94043-5272 (US)
(72) Inventor: PERIANA, Roy, A., San Jose, CA 95132 (US); TAUBE, Douglas, J., Hayward, CA 94542 (US); TAUBE, Henry, Stanford, CA 94305 (US); EVITT, Eric, R., Mountain View, CA 94040 (US)
(74) Representative: Goldin, Douglas Michael
(86) International application number: PCT/US92/01272
(87) International publication number: WO 92/14738

(56) References cited:
- US-A- 2 493 038
- US-A- 4 172 810
- US-A- 4 621 161
- US-A- 4 709 113
- US-A- 4 802 958
- US-A- 4 803 187
- US-A- 4 822 938
- CHEMICAL ABSTRACTS, vol. 113, no. 1, 2 July 1990, Columbus, Ohio, US; abstract no. 5472s, page 537 ;column 1 ;
- CHEMICAL ABSTRACTS, vol. 115, no. 21, 25 November 1991, Columbus, Ohio, US; abstract no. 231373h, page 865 ;column 1 ;
- JOURNAL OF THE CHEMICAL SOCIETY, CHEMICAL COMMUNICATIONS 1990 , LETCHWORTH GB pages 1049 - 1050 M VARGAFTIK ET AL 'HIGHLY SELECTIVE PARTIAL OXIDATION OF METHANE TO METHYL TRIFLUOROACETATE'
- JOURNAL OF THE AMERICAN CHEMICAL SOCIETY vol. 113, no. 2 , 16 January 1991 , WASHINGTON, DC US pages 700 - 701 A SEN ET AL 'LOW-TEMPERATURE,PALLADIUM(II) -CATALYZED,SOLUTION-PHASE OXIDATION OF METHANE TO A METHANOL DERIVATIVE'
- JOURNAL OF THE AMERICAN CHEMICAL SOCIETY vol. 109, no. 26 , 1987 , WASHINGTON, DC US pages 8109 - 8111 A SEN ET AL 'CARBON-HYDROGEN BOND ACTIVATION BY ELECTROPHILIC TRANSITION-METAL COMPOUNDS.'
- Angew. Chem. Int. Ed. Engl., Volume 17, 1978, OLAH et al., "Superacid-Catalyzed Oxygenation of Alkanes"; FP-909-921, see Section 1-7, 2 (pp. 916-929).
- RUDAKOV et al., "A New Case of Homogeneous Oxidation of Saturated Hydrocarbons by Platinum Complexes", pp 1610-1611, see full text.
- UDC Document Nos 541.128;546.96;547.21, issued 1979 (Plenum Publishing Corporation); TRET'YAKOV et al., "Rhenium Complexes: Homogeneous and Heterogeneous Catalysts for the Oxidation of Unsaturated Hydrocarbons", pp 189-91, Text and Tables.

## Description

This invention is a process for converting hydrocarbonaceous feeds, particularly lower alkanes, into their corresponding esters and optionally into various intermediates (such as alcohols) and optionally into liquid hydrocarbons. The hydrocarbonaceous feeds are oxidatively converted to oxy-esters at high selectivity and conversion and at practical reaction rates using at least catalytic amounts of certain class "B" metals and/or metal ions defined by the Pearson definition as "soft" or "borderline". Desirable catalysts comprise such metals as Pd, Tl, Pt, Hg, and Au. Mercury is most desired. If so desired, the oxy-esters may be converted to alcohols or other intermediates such as halides. The oxy-esters, alcohols, and other intermediates may optionally be converted to liquid hydrocarbons such as gasoline.

### BACKGROUND OF THE INVENTION

The countries of North America currently import significant portions of their needed liquid hydrocarbons from Asia and Africa. Natural gas is abundant on the North American continent but is often present in remote locations. Although natural gas may be liquefied and transported for subsequent use, appropriate refrigeration and compression equipment and transportation are quite expensive. Additionally, there are few economically viable technologies available for converting gaseous hydrocarbons to higher molecular weight liquid form materials. This invention includes a highly effective catalytic step useful in converting methane and other hydrocarbonaceous feeds, particularly lower alkanes, to another, more reactive form which may then be converted to normally liquid hydrocarbons.

It is generally accepted that conversion of methane into a reactive intermediate is the most difficult step in the overall conversion of methane into higher molecular weight hydrocarbons (see, for instance, A.E. Shilov and A.A. Shteinman, "Activation of Saturated Hydrocarbons by Metal Complexes in Solution", *Kinetika i Kataliz,* Vol. 18, No. 5, pp. 1129-1145, 1977).

Several documents disclose a variety of methods for activating methane to produce other higher molecular weight materials.

Mobil Oil Corporation is the assignee in several U.S. patents using sulfur or certain sulfur-containing compounds as the reactants in non-catalytic reactions with methane to produce methyl intermediates which can then be converted to higher molecular weight hydrocarbons.

US2493038 refers to the reaction of methane with sulphurtrioxide to form methanol and methane sulfonic acid. It does not disclose the use of an oxidation resistant acid.

Tret'yakov *et al,* UDC Doc. 541.128:546.96:547.21(1979) refers to the oxidation of alkanes to monochloro alkanes using ruthenium.

Olah *et al,* Angew.Chem.Int.Ed Enhl. 17: 909-931 (1978) refers to the oxygenation of alkanes using super-acids.

Kao *et al,* J.Am.Chem.Soc. 113:700-701 (1991) refers to attempts to oxidise methane to methanol using Pd (II).

In U.S. Patent No. 4,543,434, Chang teaches a process using the following steps: ${\text{CH}}_{\text{4}} {\text{+ 4S → CS}}_{\text{2}} {\text{+ 2H}}_{\text{2}} \text{S}$ 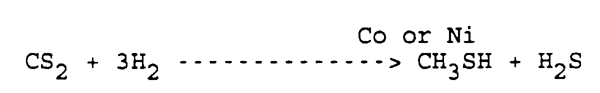 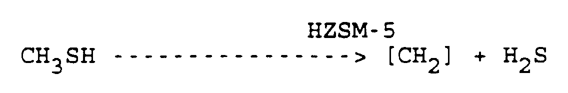 ${\text{4H}}_{\text{2}} {\text{S → 4H}}_{\text{2}} \text{+ 4S}$where "[CH₂]" is a hydrocarbon having at least two carbon atoms.

Another Mobil disclosure (U.S. Patent No. 4,864,073 to Han et al.) suggests a carbonyl sulfide-based process in which methane and carbonyl sulfide are contacted in the presence of ultraviolet light under conditions sufficient to produce CH₃SH. No other reaction initiators are said to be present. The reaction scheme is shown to be: ${\text{CH}}_{\text{4}} {\text{+ COS → CH}}_{\text{3}} \text{SH + CO}$ 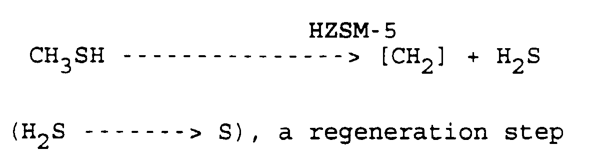 $\text{CO + S → COS}$The selectivity of the first reaction is said to be high, i.e., around 81%; however, the conversion appears to be quite low.

A disclosure similar to that in Chang is found in Mobil's U.S. Patent No. 4,864,074 to Han et al. As in Chang, the methane is contacted with sulfur. The process conditions are changed, however, so that either CS₂ or CH₃SH is formed. These sulfur compounds may then be converted in the presence of the preferred HZSM-5 zeolite catalyst to produce hydrocarbons having two or more carbon atoms. Also, as was the case with Chang, the step in which methane is converted to a methyl-sulfur compound is performed in the absence of a catalyst.

Other methods are known for producing substituted methanes which are suitable for further reaction to heavier hydrocarbons. A thermal methane chlorination process is shown in U.S. Patent No. 4,804,797 to Minet et al. A similar process is disclosed in U.S. Patent No. 3,979,470 to Firnhaber et al. although a preference for C₃ hydrocarbon feeds is expressed.

One method shown in U.S. Patent No. 4,523,040 to Olah utilizes either a solid strongly acidic catalyst or a supported Group VIII metal (particularly platinum and palladium) in the gas phase halogenation of methane to produce methyl halides. The patent indicates that monohalides are produced in 85% to 99% selectivity. Olah suggests that subsequent or concurrent catalytic hydrolysis produces methyl alcohol and/or dimethyl ether. Production of methyl oxy-esters is not shown.

The reaction of methane with palladium (II) acetate in trifluoroacetic acid to effect the trifluoroacetoxylation of methane is shown in Sen et al., "Palladium (II) Mediated Oxidative Functionalizaton of Alkanes and Arenes", *New Journal of Chemistry* (1989), Vol. 13, No. 10-11, pp. 756-760. A yield of 60% based on palladium was reported when the reaction was practiced using methane as the reactant. Consequently, the reaction with methane utilized palladium as a reactant and not as a catalyst. The extent of methane conversion, selectivity, and reaction rates were not stated.

The Sen et al. article has been criticized in Vargaftik et al., "Highly Selective Partial Oxidation of Methane to Methyl Trifluoroacetate", *Journal of the Chemical Society, Chemical Communications* (1990), pp. 1049-1050, to the extent that the results were not found to be reproducible. Vargaftik et al. discloses the catalytic oxy-esterification of methane to methyl trifluoroacetate with cobalt in trifluoroacetic acid but shows that palladium is not even suitable for stoichiometric methane oxidation in that process. With Pd, less than 0.1% yield of methyl trifluoroacetate based on palladium (II) trifluoroacetate was obtained.

The Varaftik et al. article discloses that although palladium is ineffective for the conversion of methane to methyl trifluoroacetate, Co^{III} can be used for this reaction. The Co^{III} is said to be catalytic in the presence of oxygen. The rate of the reaction was very low, 2.5 x 10⁻¹¹ mol/cc sec, (or four to five orders of magnitude away from typical commercial rates of about 10⁻⁶ mol/cc.sec). Only four turnovers of the Co ion were disclosed. The extent of methane conversion was not stated. In addition to Co, other metals were suggested which were said to allow stoichiometric oxidation of methane to methyl trifluoroacetate in varying yields (based on amount of metal charged): Mn (30%), Cu (0.1%), and Pb (10%).

A later publication by Sen et al ("Homogeneous Palladium (II) Mediated Oxidation of Methane", *Platinum Metals Review,* (1991), Vol 35, No. 3, pp. 126-132) discloses a catalytic system using palladium as the catalyst, peroxytrifluoroacetic acid as the oxidant, and a mixture of trifluoroacetic acid and trifluoroacetic anhydride as the solvent. The reaction rate was low (4.2 x 10⁻⁹ mol/cc.sec) and only 5.3 turnovers of Pd were observed. The extent of methane conversion and selectivity were not stated.

None of these disclosures shows a process in which a hydrocarbonaceous feed, particularly a lower alkane, is oxidized to an oxy-ester intermediate using our class of catalysts, acids, and oxidants nor do they show a process combining that step with a step in which the oxy-ester intermediates so-produced are then converted into heavier liquid hydrocarbons. These disclosures further do not show processes achieving high conversion and selectivity for such a catalytic oxidation to form oxy-esters, particularly at practical reaction rates.

### SUMMARY OF THE INVENTION

This invention is a catalytic oxidation process for the conversion of hydrocarbonaceous feeds, particularly lower alkanes, into oxy-esters which oxy-esters may be converted into functionalized intermediates such as alcohols, thiols, other esters, halides, and other such materials or may be converted into hydrocarbons (desirably in the gasoline boiling range). The functionalized intermediates may also be converted into hydrocarbons via a variety of known processes, e.g., oligomerization or the like.

The first step is catalytic and involves the contacting of a hydrocarbonaceous feed (particularly a lower alkane such as methane) with:
a.) particular acids,
b.) a catalyst (in at least a catalytic amount) comprising a Class "B" metal from the Mendeleev table and/or Pearson "soft" and "borderline" metal cations,
c.) and one or more specific oxidizing agents.

The Class "B" metals are as described in "Inorganic Chemistry", C.S.G. Phillips and R.S.P. Williams, Oxford University Press, 1966 at pp. 459-477. The so-called Pearson definitions of "soft" and "borderline" metal ions used in this disclosure may be found at pages 276 to 290 of "Inorganic Chemistry", James E. Huheey, Harper and Row Publishers, Second Edition, 1978. The reaction may take place at low pressures and temperatures. The preferred metal catalysts are selected from Pd, Tl, Pt, Hg, and Au; most preferred is Hg. The preferred oxidizing agents are O₂, H₂SO₄, SO₃, HNO₃, and H₂SeO₄. When a lower (eg C₁₋₄) alkane is selected as a feed, the alkane is converted to an alkyl oxy-ester of the acid which is relatively inert to further oxidation under the reaction conditions.

The oxy-esters produced in the first step may then be converted to alcohols or to other suitable intermediates. This step may be used to regenerate the acid for recycle and reuse in the first step.

The alcohols or other intermediates may then be converted to higher hydrocarbons, preferably suitable for direct use as a fuel but at least suitable for further processing to higher hydrocarbons or chemicals. Of course, the alcohols may themselves be used directly as a fuel.

### DESCRIPTION OF THE INVENTION

As noted above, this invention includes both the overall process for producing functionalized intermediates such as alcohols, thiols, other esters, halides, and other such functionalized materials or higher hydrocarbons as well as the individual step of esterifying hydrocarbonaceous feeds, particularly lower alkanes, into oxy-esters.

By the term "hydrocarbonaceous feed", we mean any molecule containing a C-H bond which is selectively oxidized to an oxy-ester using the process of this invention. These molecules may include aromatics, alkanes, alkenes, and alkynes as well as alkylaromatics and alkenyl aromatics. The hydrocarbonaceous feeds may be substituted if the substituent is stable in the reaction conditions specified by the invention. For instance, substituent groups such as alcohols, thiols, ethers, amines, carboxylic acids, and other like moieties may be present on the molecule.

The overall process, using methane as an example of a hydrocarbonaceous feed, may be outlined in the following fashion: 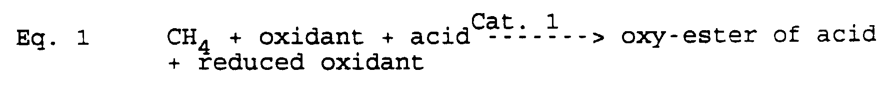 $\text{Eq. 2 oxy-ester of acid + nucleophile → functionalized intermediate + acid}$ 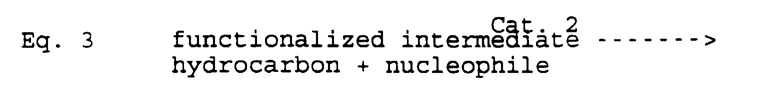 ${\text{Net Reaction: CH}}_{\text{4}} \text{+ oxidant → hydrocarbon + reduced oxidant}$

### First Step

The first step (utilizing the reaction of Eq. 1) involves contacting methane or other hydrocarbonaceous feed with an acid and an oxidizing agent in the presence of a catalyst.

The acid HX (where X is the acid's anion) may be an organic or inorganic acid such as HNO₃, H₂SO₄, CF₃CO₂H, CF₃SO₃H, H₃PO₄, H₂SeO₄, HBr, HCl, HF, HPA's (heteropolyacids), B(OH)₃ or the like, anhydrides and acid salts of these acids such as H₄P₂O₇, H₂S₂O₇ or the like, and mixtures of two or more of these acids or these acids and anhydrides.

The preferred acids are strong inorganic acids (pKa < 3.0) and especially preferred are H₂SO₄, H₄P₂O₇, and CF₃SO₃H. For greatest efficiency the acids should be oxidation resistant: they should not be oxidized by the catalyst metal in the noted reaction medium. If such an oxidation were to occur, it would obviously result in destruction of the acid and need for additional acid processing. In addition to acting as a reactant, the acid may also be used in excess, and, in some cases as an oxidant, thereby acting as reactant (to form the oxy-ester), solvent, and sometimes, oxidant.

The oxidizing agent should be selected from the group of O₂, H₂SO₄, SO₃, HNO₃, and H₂SeO₄. Although other oxidants are likely suitable under appropriate conditions, these are specifically capable of oxidizing the reduced form of the metal catalysts. Oxygen is preferred because of its ready availability and low cost. H₂SO₄ and SO₃ are desirable oxidants since the regeneration of the SO₂ produced is well known technology. The oxidant may be present in its entirety at the beginning of the reaction or (if a batch reaction is anticipated) added to the reaction as required. For instance, where H₂SO₄ is the solvent and SO₃ is the oxidant, the SO₃ is preferably added in a controlled manner to reoxidize the reduced catalyst. O₂ may be used as an oxidant in a similar fashion.

Where O₂ is the oxidant, the manner of accomplishing Eq. 1 is as follows: ${\text{Eq 1a CH}}_{\text{4}} {\text{+ ½O}}_{\text{2}} {\text{+ HX → CH}}_{\text{3}} {\text{X + H}}_{\text{2}} \text{O}$Where H₂SO₄ is both the oxidant and the acid, the process for accomplishing Eq. 1 is as follows: ${\text{Eq 1b CH}}_{\text{4}} {\text{+ 2H}}_{\text{2}} {\text{SO}}_{\text{4}} {\text{→ CH}}_{\text{3}} {\text{OSO}}_{\text{3}} {\text{H + 2H}}_{\text{2}} {\text{O + SO}}_{\text{2}}$Where SO₃ is the oxidant and H₂SO₄ is the acid, the process for accomplishing the reaction of Eq. 1 is as follows: ${\text{Eq 1c CH}}_{\text{4}} {\text{+ SO}}_{\text{3}} {\text{+ H}}_{\text{2}} {\text{SO}}_{\text{4}} {\text{→ CH}}_{\text{3}} {\text{OSO}}_{\text{3}} {\text{H + SO}}_{\text{2}} {\text{+ H}}_{\text{2}} \text{O}$Where O₂ is the oxidant and CF₃SO₃H is the acid, the process for accomplishing the reaction of Eq. 1 is as follows: ${\text{Eq 1d CH}}_{\text{4}} {\text{+ ½O}}_{\text{2}} {\text{+ CF}}_{\text{3}} {\text{SO}}_{\text{3}} {\text{H → CH}}_{\text{3}} {\text{OSO}}_{\text{2}} {\text{CF}}_{\text{3}} {\text{+ H}}_{\text{2}} \text{O}$

Since this is an oxidation process, the chemical oxidizing agent may be replaced with an electrochemical system. Where the oxidation is carried out electrochemically, the process for accomplishing the reaction of Equation 1 (at the anode) is as follows: ${\text{Eq. 1e CH}}_{\text{4}} {\text{+ HX → CH}}_{\text{3}} {\text{X + 2H}}^{\text{+}} {\text{+ 2e}}^{\text{-}}$

The catalyst used in Eq. 1 is one or more metals selected from the Class "B" metals of the Mendeleev Table and/or metals ions that are characterized as "soft" or "borderline" by the Pearson definitions. Suitable metal ions are the following: Cu, Zn, Pd, Ag, Cd, In, Sn, Sb, Te, Pt, Au, Hg, Tl, Pb, Bi, Ga, Ge, As, Po, Rh, Ir, Os, and Ru. The stable high oxidation states of these metals are characterized by a relatively high affinity for binding to "soft" polarizable ligands, e.g., CH₄. These metals bind well to organic ligands since these species are "soft" and polarizable and tend to show the following order of binding (and insolubility) to the halogens: I > Br > Cl > F. These properties are pronounced in those metal ions with electron configurations d⁵-d¹⁰ and are especially so in the heavy ions, e.g., Au^{I}, Au^{III}, Hg^{II}, Tl^{III}, Pd^{II}, Pt^{II} and Pt^{IV}. Another important characteristic of these metal ions is the relative inefficiency with which these metal ions undergo one-electron reductions while still allowing efficient two-electron reductions. For example, Hg²⁺ reduces to produce Hg₂²⁺ (a net 2e⁻ change); Hg⁺ is not a stable species and one-electron reductions with Hg²⁺ are not common. This is in contrast to metals such as Co^{III} and Mn^{III} which readily undergo 1e⁻ reduction by one-electron changes to produce the Mⁿ⁻¹ ions. Strong acids (desired for heterolytic reactions) and the corresponding anions are resistant to oxidation and more easily oxidized by one-electron than two-electron processes. Thus, the Class "B" and/or "soft" metal ions do not readily oxidize strong acid solvents or the corresponding anions but can still allow efficient reaction of methane via a two-electron heterolytic process in such media.

One such metal ion, Pd²⁺, is a powerful oxidant (E⁰ Pd²⁺/Pd⁰ = 0.98 V vs. NHE), with electronic configuation d⁸ and is characterized as a "soft" metal ion by the Pearson definition. Consistent with its classification as a "soft" metal ion and high ionization potential (19.42 eV), Pd²⁺ allows facile reactions with alkanes ("soft bases") including methane. Consistent with these "soft" properties of Pd²⁺ are the well known reactions of Pd²⁺ with arenes (palladation) to produce isolable phenyl-palladium species. These phenyl-palladium species further react to produce functionalized aryl species, including aryl esters, presumably by a similar process that occurs in the formation of methyl esters from methyl-palladium species. This metallation reaction of arenes has been relatively well studied compared to the corresponding reaction with alkanes. The initial important reaction with arenes is thought to proceed via an heterolytic electrophilic CH bond activation, Eq 4, and since this is similar to the processes proposed for reaction with alkanes (See, Periana et al and Sen et al, above), could serve as a good model for electrophilic reactions with the much more difficultly reactive alkanes, Eq 5. ${\text{Eq 4 C}}_{\text{6}} {\text{H}}_{\text{6}} {\text{+ Pd}}^{\text{2+}} {\text{→ C}}_{\text{6}} {\text{H}}_{\text{5}} {\text{-Pd}}^{\text{+}} {\text{+ H}}^{\text{+}}$${\text{Eq 5 CH}}_{\text{4}} {\text{+ Pd}}^{\text{2+}} {\text{→ CH}}_{\text{3}} {\text{-Pd}}^{\text{+}} {\text{+ H}}^{\text{+}}$

Even though Pd is acceptable from a reactivity point of view, it is expensive and the reaction rate is slow from a commercial point of view. Other more active metals would be desirable, particularly for methane activation since the goal of developing an economic process based on the heterolytic electrophilic conversion of methane to methyl oxy-esters remains an important commercial goal. Using the intrinsic chemical properties of Pd discussed above as guidelines, other class "B", "soft" metal ions with oxidation potentials >0.1 volts were identified as candidates for the conversion of methane to methyl esters: Au^{III}, Tl^{III}, Pt^{IV}, Pt^{II}, Hg^{II}, Cu^{II}, Ag^{I}, Bi^{IV}, Bi^{III}, Pb^{IV}, Pb^{II}, Rh^{III}, Sn^{IV}, Sn^{II}, Sb^{V}, Sb^{III}, Te^{IV}, Te^{III}, Ir^{III}, Ru^{III}, Ru^{IV}, Ru^{VI}, Ru^{VII}, and Ru^{VIII}. Importantly, certain of these metal ions (Tl^{III}, Au^{III}, Pt^{II}, Hg^{II}, Pb^{IV}) are known to react with arenes by electrophilic mechanisms.

We have found that Hg^{II}, Tl^{III}, Pt^{IV}, Pt^{II} Au^{I}, and Au^{III} are also effective ions for the selective oxidation of methane to methyl esters. Of these, Hg^{II} is the most effective, exhibiting much higher activity than Pd^{II}, and is significantly less expensive and is especially reactive in H₂SO₄. The stoichiometry of the reaction when using H₂SO₄ is shown in Eq 1b above.

The form in which the catalyst is introduced to the reaction medium is not particularly important; the requirements being only that it be in a form allowing oxidant, acid, and reactant access to the metal and that the form not restrict the ability of the catalytic metal to vary between oxidation states during the reaction. For instance, the metal may be introduced as a metal, salt, or complex into a liquid reaction medium. The catalytic metal (or metals) may be placed on the usual catalyst supports (carbon, zirconia, silica, alumina, etc.) provided that the supports do not interfere with the requirements listed above. We have found that introducing the metal to the liquid reaction medium in a form which produces a homogeneous catalyst is very desirable. The metal may be introduced to the liquid reaction medium in a convenient form such as the salt of the acid used in Eq. 1, although that is not required. The metallic form of the catalyst may also be used. The catalyst metal concentration must be present in at least a catalytic amount; amounts of metal ranging between 50 ppm and 1.0% by mole of the total liquid present are effective although we have found no intrinsic limitation on the metal concentration. The reaction rate is related to the concentration of the catalytic metal; higher rates result from higher metal concentration.

The esterification process conditions used in the first step are as follows:
a. temperature is greater than 50°C, preferably 50°C to 300°C, and most preferably 95°C to 200°C;
b. if the hydrocarbonaceous feed is gaseous, e.g., methane it is added at a pressure above about 0.35 MPa (50 psig), preferably above about 2.07 MPa (300 psig), and most preferably above about 3.10 MPa (450 psig); and
c. oxidant (whether pure or with other inert diluents) is added in an amount sufficient to support the reaction.
These conditions result in production of the oxy-ester of the acid in a molar amount greater than the molar amount of the catalyst metal charged in the reactor therefore giving a truly catalytic process.

We have found that the catalyst metals may also be used as reactants rather than as catalysts. If the metal is to be used as a reactant rather than as a catalyst, then substantially larger amounts of the metal may be added. For instance, using Hg(OSO₃H)₂ both as the oxidant and the catalyst in the first step: ${\text{Eq. 1f CH}}_{\text{4}} {\text{+ 2Hg(OSO}}_{\text{3}} {\text{H)}}_{\text{2}} {\text{→ CH}}_{\text{3}} {\text{OSO}}_{\text{3}} {\text{H + Hg}}_{\text{2}} {\text{(OSO}}_{\text{3}} {\text{H)}}_{\text{2}} {\text{+ H}}_{\text{2}} {\text{SO}}_{\text{4}}$then the mercury compound may be regenerated according to the following equation: ${\text{Eq. 1g Hg}}_{\text{2}} {\text{(OSO}}_{\text{3}} {\text{H)}}_{\text{2}} {\text{+ O}}_{\text{2}} {\text{→ 2Hg(OSO}}_{\text{3}} {\text{H)}}_{\text{2}} {\text{+ H}}_{\text{2}} \text{O}$

The most preferred reaction situation for accomplishing Eq. 1 above is thus: the reaction is carried out in sulfuric acid, which acts as the solvent and acid and SO₃ is the oxidant. Mercury is the catalyst.

The concentration of Hg is as discussed generically above. The Hg is typically introduced into the reaction mixtures as HgSO₄ but any form of Hg is acceptable; e.g. HgCl₂, Hg₂SO₄, Hg(NO₃)₂, Hg(CH₃)₂, Hg(C₆H₅)₂, Hg metal, Hg amalgams with various metals, etc. Although the metallic form of the metal permits a reaction to occur, the ionic form is presumed to be the catalytically active state and reactions with metallic Hg likely proceed first by oxidation of the metal to the ionic state. Importantly, metallic mercury is not observed in the reaction and the reaction remains homogeneous independent of its form when added to the reaction. The yield of the reaction when using mercury is sensitive to the concentration of the sulfuric acid and lower acid concentration results in lower yields. However, reaction does occur in 90% sulfuric acid and may occur in solutions as low as 50%. The fastest reactions and highest yields have been observed in 100% sulfuric acid. The reaction also occurs in sulfuric acid containing excess SO₃ (oleum). However, since the water generated in the reaction (Eq 1) must be removed (to prevent hydrolysis of the CH₃OSO₃H and dilution of the sulfuric acid) a preferred concentration is about 96% H₂SO₄, since this concentration can readily be maintained by removal of water through simple distillation. The reaction may also be controlled to run at any desired concentration of H₂SO₄ by continuous, controlled addition of SO₃ to the reaction mixture. This addition step prevents the build-up of water, yet allows reoxidation of the catalyst, and may allow high conversion of the H₂SO₄ to the product methyl sulfate.

It is desirable to use O₂ as the oxidant in conjunction with H₂SO₄ as the reaction medium. This reaction is shown above. This combination avoids production of SO₂ and the concomitant need for its recycle by oxidation to SO₃.

Use of devices promoting good mixing between the gas and liquid phases is desirable.

This reaction may be carried out in a molten salt reaction medium rather than in acids as solvents (so long as the stoichiometric requirements of the reaction are met), e.g. molten KHSO₄, NaHSO₄, K₂S₂O₇, Na₃BO₃, etc. Alternatively, solid acids may be used as the reacting acid, with or without an added liquid as solvent. Under these conditions the acid would be converted to a solid methyl ester which may then be treated with a nucleophile to release the methyl intermediate. These process variations have certain potential advantages such as lower corrosivity, lower volatility, increased reaction medium inertness, and lower overall cost.

### Second Step

This step is shown above as Eq. 2. It is an optional step and is carried out generally for the purpose of replacing the oxy-ester formed in the first step with an intermediate which is both reactive in the third step and does not substantially degrade the catalyst used in that later step. This step allows regeneration of the acid utilized in the first step for generation of the methyl ester.

The methyl ester may be separated from the first step reaction media by commonly practiced steps such as flashing or distillation. However, there is no need to isolate the methyl ester since solutions of the methyl ester and acid solvent react with added nucleophile to produce an intermediate which is reactive in the third step. The nucleophile in Eq. 2 is suitably then mixed with the methyl oxy-ester (pure or in the acid solvent) to produce a "methyl intermediate". By "methyl intermediate" is meant methanol, if the nucleophile is H₂O; methyl halide, if the nucleophile is a hydrogen halide such as HCl, HBr, or HI; methyl amino, if the nucleophile is NH₃; methyl thiol, if the nucleophile is H₂S; methyl or carboxylic esters if the nucleophile is CH₃CO₂H, HCO₂H,etc.; (di)-methyl sulphite if the nucleophile is H₂SO₃ or SO₂; (tri)-methyl borate if the nucleophile is B(OH)₃; (di)-methyl carbonate if the nucleophile is H₂CO₃ or CO₂; methyl amines if the nucleophile is NH₃, CH₃NH₂, etc.; or acetonitrile if the nucleophile is HCN. Other nucleophiles can be utilized and would be known to the ordinary skilled worker but should be of a type not decomposed by reaction with the acid solvent. These reactions proceed readily to completion. An excess of the nucleophile is desirable. The preferred nucleophile is H₂O since it may also be produced in the first step. The product methanol may be used directly or may be converted to a variety of hydrocarbons in a following step or steps.

In some cases the first and second step may be combined if a suitable nucleophile is selected. For example, a mixture of H₂SO₄ and acetic acid results in the formation of methyl acetate that can be isolated by distillation during the progress of the reaction thus combining steps 1 and 2. Similarly, mixtures of H₂SO₄ and HCl result in the formation of CH₃Cl.

### Third Step

This step (shown above as Eq. 3) includes conversion of the methyl intermediate to a longer chain or higher molecular weight hydrocarbon.

Suitable processes for converting methanol and other methyl intermediates to higher molecular weight hydrocarbons are found in U.S. Patent Nos. 3,894,107 and 3,979,472 to Butter et al. Butter shows the production of olefinic and aromatic compounds by contacting the methyl intermediate with an aluminosilicate catalyst, preferably HZSM-5, at a temperature between 650°F and 1000°F.

Similarly, Butter suggests a process using a preferable catalyst of antimony oxide and HZSM-5 at a temperature between 250°C and 700°C.

The ZSM-5 zeolite has been disclosed as a suitable molecular sieve catalyst for converting methyl alcohol into gasoline-range hydrocarbons. See, for instance, U.S. Patent Nos. 3,702,886 to Argauer et al. and 3,928,483 to Chang et al.

Other processes include those described in U.S. Patent No. 4,373,109 to Olah (bifunctional acid-base catalyzed conversion of methanol and other methyl intermediates into lower olefins) ; U.S. Patent No. 4,687,875 to Currie et al. (metal coordination complexes of heteropolyacids as catalysts for converting short chain aliphatic alcohols to short chain hydrocarbons) ; U.S. Patent No. 4,524,234 to Kaiser (production of hydrocarbons preferably from methanol using aluminophosphate molecular sieves); and U.S. Patent No. 4,579,996 to Font Freide et al. (production of hydrocarbons from C₁ to C₄ monohaloalkanes using layered clays); etc. Each of the above is potentially suitable for the third step of this process and their contents are incorporated by notice.

### Integrated Process

Where the process steps outlined as Eqs. 1-3 above are integrated, as might be done in an operating plant where CH₄ is the hydrocarbonaceous feed, O₂ is the oxidant, and HY is a nucleophile, the overall process scheme is as follows: ${\text{CH}}_{\text{4}} {\text{+ ½O}}_{\text{2}} {\text{+ HX → CH}}_{\text{3}} {\text{X + H}}_{\text{2}} \text{O}$${\text{CH}}_{\text{3}} {\text{X + HY → CH}}_{\text{3}} \text{Y + HX}$${\text{CH}}_{\text{3}} {\text{Y → [CH}}_{\text{2}} \text{] + HY}$${\text{Net Reaction: CH}}_{\text{4}} {\text{+ ½O}}_{\text{2}} {\text{→ [CH}}_{\text{2}} {\text{] + H}}_{\text{2}} \text{O}$Where H₂SO₄ is both the oxidant and the acid and water is the nucleophile, the process for accomplishing the overall process is as follows: ${\text{CH}}_{\text{4}} {\text{+ 2 H}}_{\text{2}} {\text{SO}}_{\text{4}} {\text{→ CH}}_{\text{3}} {\text{OSO}}_{\text{3}} {\text{H + 2 H}}_{\text{2}} {\text{O + SO}}_{\text{2}}$${\text{SO}}_{\text{2}} {\text{+ ½O}}_{\text{2}} {\text{+ H}}_{\text{2}} {\text{O → H}}_{\text{2}} {\text{SO}}_{\text{4}}$${\text{CH}}_{\text{3}} {\text{OSO}}_{\text{3}} {\text{H + H}}_{\text{2}} {\text{O → CH}}_{\text{3}} {\text{OH + H}}_{\text{2}} {\text{SO}}_{\text{4}}$${\text{CH}}_{\text{3}} {\text{OH → [CH}}_{\text{2}} {\text{] + H}}_{\text{2}} \text{O}$${\text{Net Reaction: CH}}_{\text{4}} {\text{+ ½O}}_{\text{2}} {\text{→ [CH}}_{\text{2}} {\text{] + H}}_{\text{2}} \text{O}$ It should be understood that the first two reactions expressed immediately above are equivalent in sum to Eq. 1 of the overall process.

Where SO₃ is the oxidant, H₂SO₄ is the acid, and water is the nucleophile, the process for accomplishing the overall process is as follows: ${\text{CH}}_{\text{4}} {\text{+ SO}}_{\text{3}} {\text{+ H}}_{\text{2}} {\text{SO}}_{\text{4}} {\text{→ CH}}_{\text{3}} {\text{OSO}}_{\text{3}} {\text{H + SO}}_{\text{2}}$${\text{SO}}_{\text{2}} {\text{+ ½O}}_{\text{2}} {\text{→ SO}}_{\text{3}}$${\text{CH}}_{\text{3}} {\text{OSO}}_{\text{3}} {\text{H + H}}_{\text{2}} {\text{O → CH}}_{\text{3}} {\text{OH + H}}_{\text{2}} {\text{SO}}_{\text{4}}$${\text{CH}}_{\text{3}} {\text{OH → [CH}}_{\text{2}} {\text{] + H}}_{\text{2}} \text{O}$${\text{Net Reaction: CH}}_{\text{4}} {\text{+ ½O}}_{\text{2}} {\text{→ [CH}}_{\text{2}} {\text{] + H}}_{\text{2}} \text{O}$It should be understood that the first two reactions expressed immediately above are equivalent in sum to Eq. 1 of the overall process.

Where O₂ is the oxidant, CF₃SO₃H is the acid, and water is the nucleophile, the process is as follows: ${\text{CH}}_{\text{4}} {\text{+ ½O}}_{\text{2}} {\text{+ CF}}_{\text{3}} {\text{SO}}_{\text{3}} {\text{H → CH}}_{\text{3}} {\text{OSO}}_{\text{2}} {\text{CF}}_{\text{3}} {\text{+ H}}_{\text{2}} \text{O}$${\text{CH}}_{\text{3}} {\text{OSO}}_{\text{2}} {\text{CF}}_{\text{3}} {\text{+ H}}_{\text{2}} {\text{O → CH}}_{\text{3}} {\text{OH + CF}}_{\text{3}} {\text{SO}}_{\text{3}} \text{H}$${\text{CH}}_{\text{3}} {\text{OH → [CH}}_{\text{2}} {\text{] + H}}_{\text{2}} \text{O}$${\text{Net Reaction: CH}}_{\text{4}} {\text{+ ½O}}_{\text{2}} {\text{→ [CH}}_{\text{2}} {\text{] + H}}_{\text{2}} \text{O}$These reaction schemes permit regeneration and recycle of the acid and, in some instances, the auxiliary oxidant, which makes the process more economical. Some acids require additional steps to separate the nuecleophile but such steps are known to the ordinarily skilled worker.

### EXAMPLES

These examples are intended to show portions of the overall inventive process, in particular the esterification reaction utilizing methane as the reactant. The remainder of the process steps are easily selectable from known processes.

### Example 1

This example shows the use of a palladium catalyst, SO₃ as oxidant, and H₂SO₄ as the acid in esterifying methane.

A 100 ml glass reactor having an integral heater-stirrer was loaded with 15 ml of oleum (20% SO₃) and 0.0304 gm PdSO₄ · 2H₂O (1.27x10⁻⁴ mol). The system was purged with N₂ and pressured to 0.69 MPa (100 psig) with CH₄. The stirrer and heater were started. During heat-up, the pressure was adjusted to maintain about 0.69 MPa (100 psig). The reaction was carried out at about 100°C and 0.69 MPa (100 psig). After about three hours, the reactor was cooled to about 25°C, the pressure bled down, and samples of the gaseous effluent taken. Samples of the reactor solution (in range of color from orange to red without black precipitate) were also taken.

The gases in the reactor were analyzed using a gas chromatograph and the gas remaining in the reactor contained CH₄, 2.96x10⁻⁴ mols carbon dioxide and 9.28x10⁻³ mols SO₂.

The reaction produced a large amount of SO₂ caused by the reoxidation of Pd⁰. The yield of the methyl ester CH₃OSO₃H was 3.6X10⁻³ mols as determined by saponification of the reaction samples and subsequent analysis of the released CH₃OH. This shows a 2834% yield on palladium and verifies that palladium is catalytic in the process. The yield of methyl ester was also confirmed by H-NMR (using internal standards in coaxial tubes).

### Example 2

This example demonstrates the use of H₂SO₄ both as an oxidant and as esterfying acid in the reaction.

The process of Example 1 was repeated except that SO₃ was omitted. An amount of 8.5 ml of H₂SO₄ was used both as the acid and oxidant. The PdSO₄ · 2H₂O was added in an amount of 0.0035 gm (1.5x10⁻⁵ mol).

The gas chromatograph data showed 6.64x10⁻⁴ mols of SO₂ in the reactor along with 2.85x10⁻⁴ mols carbon dioxide. The yield of CH₃OSO₃H was 1.46x10⁻⁴ mols or a 970% yield on the amount of palladium present in the reactor. The reaction was catalytic in the palladium metal.

### Example 3

This example shows the use of O₂ as the oxidant, H₂SO₄ as the esterifying acid, and palladium as the catalyst. In addition, the process is a continuous feed operation rather than a batch operation as shown in Examples 1 and 2.

A 150 ml TEFLON-lined reactor having an integral heater-stirrer was loaded with 20 ml of H₂SO₄ and 0.478 gm (2x10⁻³ mol) PdSO₄ · 2H₂O. The system was purged with N₂ and pressured to 4.14 MPa (600 psig) with CH₄. The stirrer and heater were started. The reaction was carried out at 175°C. The effluent gas was continuously sampled. The feed gases were introduced at 20 cc/minute of O₂ (8.9x10⁻⁴ mol/minute) and 80 cc/minute of CH₄ (3.6x10⁻³ mol/minute).

At about two hours, the molar ratio of CH₄/O₂ stabilized at about 4/1 and the system pressure stabilized at 4.31 MPa (625 psig). After about six hours the reaction was terminated by cooling the reactor to 50°C.

Gas chromatograph analysis of the gases showed that, on the average, 7.59x10⁻³ mols of carbon dioxide and 9.28x10⁻⁴ mols of SO₂ were produced. The yield of CH₃OSO₃H was 5.0x10⁻³ mols for a yield of 250% based on the amount of palladium present.

Again, this demonstrates the palladium metal to be catalytic in this process.

### Example 4

This example shows the catalytic esterification of methane using CF₃SO₃H (triflic acid) as the acid, O₂ as the oxidant, and palladium as the catalyst.

A 50 ml Hastalloy C reactor equipped with a stirrer was loaded with 30 ml of triflic acid, 0.2 ml H₂O, and 0.25 g of palladium black (Pd⁰). The reactor was pressured to 3.45 MPa (500 psig) with pure O₂, heated to 180°C, and stirred for two hours to convert the palladium black to the catalytically active form.

The reactor was cooled and purged with N₂ to remove the O₂. The reactor was pressured to 6.89 MPa (1000 psig) with CH₄. A flow of 200 standard cc/minute of CH₄ was then established. The gas leaving the reactor was scrubbed using aqueous Na₂CO₃. A flow of 50 standard cc/minute of O₂ was then introduced, the reactor heated to 180°C, and stirring commenced. The exiting gases were periodically analyzed for carbon dioxide and other product gases. The reaction was terminated at three hours by stopping the gas flows and allowing the reactor to cool to room temperature. The reactor head space was vented through the Na₂CO₃ trap. The contents of the reactor, Na₂CO₃ trap, and the exit gases were analyzed for methyl triflate, methanol, carbon dioxide, and other products.

The only carbon-containing products were methanol, methyl triflate, and carbon dioxide. Less than 5% of the methane consumed was converted to carbon dioxide. The sum of the amounts of methyl ester (methyl triflate) found in the reactor and methanol found in the Na₂CO₃ trap (due to *in situ* saponification) was calculated to produce a yield of 500% based on palladium.

### Example 5

This is a comparative example which shows the need for an oxidant in the process in order to obtain catalysis with respect to palladium. Specifically, methane was used as the lower alkane, CF₃SO₃H (triflic acid) as the acid, and palladium as the oxidant.

A 50 ml Hastalloy C reactor equipped with a stirrer was loaded with 30 ml of triflic acid (CF₃SO₃H), 0.2 ml H₂O, and 0.25 g of palladium black (Pd⁰). The reactor was pressured to 500 psig with pure O₂, heated to 180°C, and stirred for two hours to convert the palladium black to the oxidant.

The reactor was cooled and purged with N₂ to remove the O₂. The reactor was pressured to 4.14 MPa (600 psig) with CH₄. In Example 4, 50 cc/minute of O₂ was introduced into the reactor; oxygen was not included in this example. The reactor was heated to 180°C, the pressure adjusted to 6.89 MPa (1000 psig) with CH₄ and stirring commenced.

The reaction was terminated at three hours by allowing the reactor to cool to room temperature. The reactor head space was vented through a Na₂CO₃ trap. The contents of the reactor, Na₂CO₃ trap, and the exit gases were analyzed for methyl triflate, methanol, carbon dioxide, and other products.

The only carbon-containing products were methanol, methyl triflate, and carbon dioxide. Only trace amounts of carbon dioxide were detected. The sum of the amounts of methyl ester (methyl triflate) found in the reactor and methanol found in the Na₂CO₃ trap (due to *in situ* saponification) provided a yield of 92% based on palladium. Without the added oxidant (such as O₂ as used in Example 4) the palladium was not catalytic.

### Example 6

The following examples (A. through I.) show the catalytic effect of mercury in our inventive process.
A. A 50 ml high pressure reactor was charged with 25 ml of 100% H₂SO₄ and 2.0 g of HgSO₄ (270 mM). The contents of the reactor were stirred, the reactor was flushed with methane and heated to 188°C under 12.4 MPa (1800 psig) of methane. After 1 hr the reactor was cooled to room temperature. A gas sample of the gas in the reactor was obtained for gas chromatographic analysis and an aliquot of the reaction mixture was qualitatively analyzed for methyl sulfate by ¹³C NMR and ¹H NMR. A second aliquot was first diluted with 3 volumes of water and the resulting solution heated in a sealed container for 2 hrs. The resulting solution was quantitatively analyzed for free CH₃OH by HPLC using-an ion exclusion column with an eluant of 0.01% H₂SO₄ in H₂O and a refractive index detector. Based on the high pressure liquid chromatography ("HPLC") and nuclear magnetic resonance ("NMR") analyses, the yield of methyl sulfate was 746 mol% (25 mmoles, 1008 mM, Table I, entry 1) based on HgSO₄ added (assuming that the reaction proceeds via the Hg redox couple Hg²⁺/Hg₂²⁺). This corresponds to a productivity rate of 2.8 x 10⁻⁷ mol. of oxy-ester/cc of reaction volume - sec based on the conversion of methane to methyl sulfate. Trace levels of acetic acid and dimethylether were detected. Gas chromatographic analysis showed that copious amounts of SO₂ and trace levels of CO₂ were produced.
B. To show the effect of HgSO₄ concentration, the general procedure of example A was repeated both with 1000 mM and 100 mM of HgSO₄ for 60 minutes under 3.52 MPa (510 psig) of methane. The results shown in Table I (entries 2 and 3) demonstrate that the higher concentrations of HgSO₄ result in higher yields of methyl sulfate over the same reaction time.
C. To show the greater effectiveness of Hg^{II} as compared to Pd^{II}, the general procedure of example A was repeated but with 165 mM of PdSO₄ and 100 mM HgSO₄ for 180 minutes under 2.76 MPa (400 psig) of methane. The results shown in Table I (entries 4 and 5) demonstrate that, under identical conditions, the HgSO₄ system is about 17 times more active than the PdSO₄ system for the production of methyl sulfate.
D. As a "blank" run, the procedure of Example C was repeated but without added PdSO₄ or HgSO₄. As may be seen from the results in Table I (entry 6) no methyl sulfate was produced in the reaction.
E. To show the effect of acid concentration, the general procedure of Example A was twice repeated but with 90% (wt) H₂SO₄ (remainder H₂O) and 100% (wt) H₂SO₄, 100 mM HgSO₄, and 3.52 to 3.86 MPa (510 to 560 psig) of methane for two hours. The results shown in Table I (entries 7 and 8) demonstrate that the yield of methyl sulfate was 464 mol% based on HgSO₄ added in the 100% H₂SO₄ and 155 mol% in the 90% H₂SO₄ system.
F. To show the effect of temperature the general procedure of Example A was repeated at 140°C, 160°C, 180°C and 200°C with 100mM HgSO₄ for two hours under 3.52 MPa (510 psig) of methane. The results shown in Table I (entries 8, 9, 10 and 11) demonstrate that increasing temperatures from 140°-180° lead to increased yields of methyl sulfate. At 200°C, a loss in yield was observed.
G. To show the effect of methane pressure, the general procedure of Example A was repeated with 100mM HgSO₄ at methane pressures from 2.48 to 12.6 MPa (360 psig to 1820 psig). The results shown in Table I (entries 12 and 13) demonstrate that the yield of methyl sulfate increases at higher pressures.
H. To show that the reaction proceeds in other acids, the general procedure of Example A was repeated in pyrophosphoric acid and trifluoromethanesulfonic acids. The results shown in Table I (entries 14 and 15) demonstrate that the reaction can be successfully carried out in pyrophosphoric acid and trifluoromethanesulfonic acids.
I. To show that the reaction proceeds with different forms of Hg, the general procedure of Example A was repeated variously using HgO, HgCl₂, Hg₂SO₄, and Hg metal in place of HgSO₄. The results shown in Table I (entries 16, 17, 18, and 19) demonstrate that these forms of Hg also leads to formation of methyl esters.

**TABLE I**

| Entry | Metal Salt | [Metal] (mM) | Acid | Temp (°C) | Rxn Time (min) | [CH₄] (MPa) (psig) | [CH₃OH] (mM) |
|---|---|---|---|---|---|---|---|
| 1 | HgSO₄ | 270 | H₂SO₄ (100%) | 180 | 60 | 12.4 (1800) | 1008 |
| 2 | HgSO₄ | 100 | H₂SO₄ (100%) | 180 | 60 | 3.52 (510) | 247 |
| 3 | HgSO₄ | 1000 | H₂SO₄ (100%) | 180 | 60 | 3.52 (510) | 378 |
| 4 | PdSO₄ | 165 | H₂SO₄ (100%) | 180 | 180 | 2.76 (400) | 50 |
| 5 | HgSO₄ | 100 | H₂SO₄ (100%) | 180 | 180 | 2.76 (400) | 526 |
| 6 | -- | -- | H₂SO₄ (100%) | 180 | 180 | 2.76 (400) | 0 |
| 7 | HgSO₄ | 100 | H₂SO₄ (90%) | 180 | 120 | 3.86 (560) | 155 |
| 8 | HgSO₄ | 100 | H₂SO₄ (100%) | 180 | 120 | 3.52 (510) | 464 |
| 9 | HgSO₄ | 100 | H₂SO₄ (100%) | 140 | 120 | 3.52 (510) | 16 |
| 10 | HgSO₄ | 100 | H₂SO₄ (100%) | 160 | 120 | 3.52 (510) | 121 |
| 11 | HgSO₄ | 100 | H₂SO₄ (100%) | 200 | 120 | 3.52 (510) | 452 |
| 12 | HgSO₄ | 100 | H₂SO₄ (100%) | 180 | 60 | 2.48 (360) | 187 |
| 13 | HgSO₄ | 100 | H₂SO₄ (100%) | 180 | 60 | 12.6 (1820) | 394 |
| 14 | HgSO₄ | 100 | H₄P₂O₇ (100%) | 220 | 114 | 2.76 (400) | 26 |
| 15 | HgO | 230 | CF₃SO₃H (100%) | 180 | 120 | 6.21 (900) | 112 |
| 16 | HgO | 155 | H₂SO₄ (100%) | 180 | 70 | 6.41 (930) | 175 |
| 17 | HgCl₂ | 100 | H₂SO₄ (100%) | 180 | 180 | 6.21 (900) | 16 |
| 18 | HgO | 155 | H₂SO₄ (100%) | 180 | 180 | 352 (510) | 200 |
| 19 | Hg₂SO₄ | 51 | H₂SO₄ (100%) | 180 | 180 | 6.34 (920) | 609 |

### Example 7

This example demonstrates that other class "B" metals and/or Pearson "soft" metal ions can be utilized in our process for the selective oxidation of methane, the general procedure of Example 6 (entry A.) was repeated variously using the metals shown in Table II (entries 1 - 4). The metals Tl, Pt, and Au successfully facilitated the oxidation of methane to the methyl esters. Although the molar amount of methyl ester produced in entries 1 - 4 was less than the amount of metal present in the reaction medium (the metal could therefore be considered either a reactant or a catalyst), it as apparent that the use of oxygen to oxidize these metals would result in a process utilizing the metals in a truly catalytic fashion.

**Table II**

| Entry | Metal Salt | [Metal] (mM) | Acid | Temp (°C) | Rxn Time (min) | [CH₄] (MPa) (psig) | [CM₃OH] (mM) |
|---|---|---|---|---|---|---|---|
| 1 | TI₂O₃ | 100 | CF₃SO₃H (100%) | 170 | 120 | .3.45 (500) | 123 |
| 2 | TI₂O₃ | 50 | H₂SO₄ (100%) | 180 | 180 | 2.76 (400) | 38 |
| 3 | PtO₂ | 62 | H₂SO₄ (100%) | 180 | 185 | 2.76 (400) | 48 |
| 4 | Au(OH)₃ | 100 | H₂SO₄ (100%) | 180 | 120 | 6.34 (920) | 94 |

The invention has been shown both by description and by example. The examples are only examples; they should not be used in any fashion to limit the scope of the invention otherwise described here.

## Claims

1. A process for converting a hydrocarbonaceous feed to higher molecular weight hydrocarbons, the process comprising:
(a) contacting one or more hydrocarbonaceous feeds; an oxidizing agent which is HNO₃, O₂, SO₃, H₂SO₄, or H₂SeO₄; an oxidation resistant acid with a pKa < 3.0; and
a catalyst in at least a catalytic amount comprising one or more of the metals Cu, Zn, Pd, Ag, Cd, In, Sn, Sb, Te, Pt, Au, Hg, Tl, Pb, Bi, Ga, Ge, As, Po, Rh, Ir, Os or Ru or a cation thereof:
under esterification conditions to produce an oxy-ester of the acid in a molar amount greater than the molar amount of the catalyst;
(b) converting the oxy-ester of the acid to a functional intermediate, and
(c) catalytically converting the functional intermediate to higher molecular weight hydrocarbons.

2. A process according to claim 9 where the hydrocarbonaceous feed comprises aromatics, alkanes, alkenes, alkynes, alkylaromatics and alkenyl aromatics.

3. A process according to claim 1 or 2 where the hydrocarbonaceous feed contains substituent groups selected from alcohols, thiols, ethers, amines and carboxylic acid moieties.

4. A process according to any preceding claim for converting C₁₋₄ alkanes to higher molecular weight hydrocarbons, the process comprising:
(a) contacting one or more C₁₋₄ alkanes;
an oxidizing agent which is HNO₃, O₂, SO₃, H₂SO₄, or H₂SeO₄;
an oxidation resistant acid with a pKa < 3.0; and
a catalyst in at least a catalytic amount comprising one or more of the metals Cu, Zn, Pd, Ag, Cd, In, Sn, Sb, Te, Pt, Au, Hg, Tl, Pb, Bi, Ga, Ge, As, Po, Rh, Ir, Os or Ru or a cation thereof:
under esterification conditions to produce an alkyl oxy-ester of the acid in a molar amount greater than the molar amount of the catalyst;
(b) converting the alkyl oxy-ester of the acid to an alkyl intermediate; and
(c) catalytically converting the alkyl intermediate to higher molecular weight hydrocarbons.

5. A process for esterifying one or more C₁₋₄ alkanes, the process comprising:
(a) contacting the one or more C₁₋₄ alkanes;
one or more of the oxidizing agents HNO₃, O₂, SO₃, H₂SO₄, or H₂SeO₄;
an oxidation resistant acid with a pKa < 3.0; and
a catalyst in at least a catalytic amount comprising one or more of the metals Cu, Zn, Pd, Ag, Cd, In, Sn, Sb, Te, Pt, Au, Hg, Tl, Pb, Bi, Ga, Ge, As, Po, Rh, Ir, Os or Ru or a cation thereof;
under esterification conditions to produce an alkyl oxy-ester of the acid in a molar amount greater than the molar amount of the catalyst; and
(b) recovering the alkyl oxy-ester of the acid.

6. A process according to claim 4 or 5 where the alkane comprises methane.

7. A process according to claim 6 where the alkyl intermediate comprises methanol.

8. A process according to claim 4 or 5 where the alkane comprises ethane, propane or butane.

9. A process according to any preceding claim where the oxidation is conducted at the anode of an electrochemical cell.

10. A process according to any preceding claim where the oxidizing agent is O₂.

11. A process according to any of claims 1 to 9 where the oxidizing agent is SO₃.

12. A process according to any of claims 1 to 9 where the oxidizing agent is H₂SO₄.

13. A process according to any preceding claim where the acid is a solid acid.

14. A process according to any preceding claim where the acid is HF, HCl, HBr, H₂SeO₄, HNO₃, H₂SO₄, CF₃CO₂H, CF₃SO₃H, H₃PO₄, (heteropolyacids), B(OH)₃, an anhydride or acid salt of these acids, and mixtures of two or more of these acids or these acids and anhydrides.

15. A process according to claim 14 where the acid is H₂SO₄.

16. A process according to claim 14 where the acid is H₄P₂O₇.

17. A process according to claim 14 where the acid is CF₃SO₃H.

18. A process according to any preceding claim where the catalytic metal is Pd, Tl, Pt, Hg or Au, or a cation thereof.

19. A process according to claim 18 where the catalytic metal is Hg or a cation thereof.

20. A process according to any preceding claim wherein step (a) takes place in the presence of the alkyl oxy-ester conversion step in (b) so that the alkyl oxy-ester is simultaneously converted to the alkyl intermediate without isolation of the alkyl oxy-ester.

## Patentansprüche

1. Verfahren zum Umwandeln einer kohlenwasserstoffhaltigen Beschickung in Kohlenwasserstoffe mit einem größeren Molekulargewicht, wobei das Verfahren umfaßt:
(a) Inkontaktbringen von einem oder mehreren kohlenwasserstoffhaltigen Beschickungen;
eines Oxidationsmittels, welches HNO₃, O₂, SO₃, H₂SO₄ oder H₂SeO₄ ist;
einer oxidationsbeständigen Säure mit einem pKa < 3,0; und
einer mindestens katalytischen Menge eines Katalysators, umfassend eines oder mehrere der Metalle Cu, Zn, Pd, Ag, Cd, In, Sn, Sb, Te, Pt, Au, Hg, Tl, Pb, Bi, Ga, Ge, As, Po, Rh, Ir, Os oder Ru oder ein Kation hiervon:
unter Veresterungsbedingungen, um ein Oxyester der Säure zu erzeugen, in einer molaren Menge, größer als die molare Menge des Katalysators;
(b) Umwandeln des Oxyesters der Säure in eine funktionelle Zwischenverbindung, und
(c) katalytisches Umwandeln der funktionellen Zwischenverbindung in Kohlenwasserstoffe mit einem höheren Molekulargewicht.

2. Verfahren gemäß Anspruch 1, wobei die kohlenwasserstoffhaltige Beschickung Aromaten, Alkane, Alkene, Alkine, Alkylaromaten und Alkenylaromaten umfaßt.

3. Verfahren gemäß Anspruch 1 oder 2, wobei die kohlenwasserstoffhaltige Beschickung Substituenten enthält ausgewählt aus Alkohol-, Thiol-, Ether-, Amin- und Carbonsäuregruppen.

4. Verfahren gemäß einem der vorstehenden Ansprüche, um C₁₋ ₄-Alkane in Kohlenwasserstoffe mit einem höheren Molekulargewicht umzuwandeln, wobei das Verfahren umfaßt:
(a) Inkontaktbringen von einem oder mehreren C₁₋₄-Alkanen;
eines Oxidationsmittels, welches HNO₃, O₂, SO₃, H₂SO₄ oder H₂SeO₄ ist;
einer oxidationsbeständigen Säure mit einem pKa < 3,0; und
einer mindestens katalytischen Menge eines Katalysators, umfassend eines oder mehrere der Metalle Cu, Zn, Pd, Ag, Cd, In, Sn, Sb, Te, Pt, Au, Hg, Tl, Pb, Bi, Ga, Ge, As, Po, Rh, Ir, Os oder Ru oder ein Kation hiervon:
unter Veresterungsbedingungen, um ein Alkyloxyester der Säure zu erzeugen, in einer molaren Menge, größer als die molare Menge des Katalysators;
(b) Umwandeln des Alkyloxyesters der Säure in eine Alkylzwischenverbindung, und
(c) katalytisches Umwandeln der Alkylzwischenverbindung in Kohlenwasserstoffe mit einem höheren Molekulargewicht.

5. Verfahren zur Veresterung von einem oder mehreren C₁₋₄-Alkanen, wobei das Verfahren umfaßt:
(a) Inkontaktbringen von einem oder mehreren C₁₋₄-Alkanen;
eines oder mehrere der Oxidationsmittel HNO₃, O₂, SO₃, H₂SO₄ oder H₂SeO₄;
einer oxidationsbeständigen Säure mit einem pKa < 3,0; und
einer mindestens katalytischen Menge eines Katalysators, umfassend eines oder mehrere der Metalle Cu, Zn, Pd, Ag, Cd, In, Sn, Sb, Te, Pt, Au, Hg, Tl, Pb, Bi, Ga, Ge, As, Po, Rh, Ir, Os oder Ru oder ein Kation hiervon:
unter Veresterungsbedingungen, um ein Alkyloxyester der Säure zu erzeugen, in einer molaren Menge, größer als die molare Menge des Katalysators; und
(b) Wiedergewinnen des Alkyloxyesters der Säure.

6. Verfahren gemäß Anspruch 4 oder 5, wobei das Alkan Methan umfaßt.

7. Verfahren gemäß Anspruch 6, wobei die Alkylzwischenverbindung Methanol umfaßt.

8. Verfahren gemäß Anspruch 4 oder 5, wobei das Alkan Ethan, Propan oder Butan umfaßt.

9. Verfahren gemäß einem der vorstehenden Ansprüche, wobei die Oxidation an der Anode einer elektrochemischen Zelle durchgeführt wird.

10. Verfahren gemäß einem der vorstehenden Ansprüche, wobei das Oxidationsmittel O₂ ist.

11. Verfahren gemäß einem der Ansprüche 1 bis 9, wobei das Oxidationsmittel SO₃ ist.

12. Verfahren gemäß einem der Ansprüche 1 bis 9, wobei das Oxidationsmittel H₂SO₄ ist.

13. Verfahren gemäß einem der vorstehenden Ansprüche, wobei die Säure eine feste Säure ist.

14. Verfahren gemäß einem der vorstehenden Ansprüche, wobei die Säure HF, HCl, HBr, H₂SeO₄, HNO₃, H₂SO₄, CF₃CO₂H, CF₃SO₃H, H₃PO₄, (Heteropolysäuren), B(OH)₃, ein Anhydrid oder Säuresalz einer dieser Säuren, Gemische von zwei oder mehreren dieser Säuren oder dieser Säuren und Anhydride ist.

15. Verfahren gemäß Anspruch 14, wobei die Säure H₂SO₄ ist.

16. Verfahren gemäß Anspruch 14, wobei die Säure H₄P₂O₇ ist.

17. Verfahren gemäß Anspruch 14, wobei die Säure CF₃SO₃H ist.

18. Verfahren gemäß einem der vorstehenden Ansprüche, wobei das katalytische Metall Pd, Tl, Pt, Hg oder Au oder ein Kation hiervon ist.

19. Verfahren gemäß Anspruch 18, wobei das katalytische Metall Hg oder ein Kation hiervon ist.

20. Verfahren gemäß einem der vorstehenden Ansprüche, wobei Schritt (a) in Gegenwart des Alkyloxyesterkonversionsschrittes von Schritt (b) stattfindet, so daß der Alkyloxyester gleichzeitig ohne Isolation des Alkyloxyesters in die Alkylzwischenverbindung umgewandelt wird.

## Revendications

1. Procédé pour convertir une charge hydrocarbonée en hydrocarbures de plus haut poids moléculaire, le procédé comprenant les étapes consistant à :
(a) mettre en contact une ou plusieurs charges hydrocarbonées ;
un agent oxydant qui est HNO₃, O₂, SO₃, H₂SO₄ ou H₂SeO₄ ;
un acide résistant à l'oxydation, ayant un pKa < 3,0 ; et
un catalyseur, au moins en une quantité catalytique, comprenant un ou plusieurs des métaux Cu, Zn, Pd, Ag, Cd, In, Sn, Sb, Te, Pt, Au, Hg, Tl, Pb, Bi, Ga, Ge, As, Po, Rh, Ir, Os ou Ru ou un cation de ceux-ci ;
dans des conditions d'estérification pour produire un oxyester de l'acide en une quantité molaire plus grande que la quantité molaire du catalyseur ;
(b) convertir l'oxyester de l'acide en un produit intermédiaire fonctionnel, et
(c) convertir catalytiquement le produit intermédiaire fonctionnel en hydrocarbures de plus haut poids moléculaire.

2. Procédé selon la revendication 1, dans lequel la charge hydrocarbonée comprend des produits aromatiques, des alcanes, des alcènes, des alcynes, des produits alkyl-aromatiques et alcénylaromatiques.

3. Procédé selon la revendication 1 ou 2, dans lequel la charge hydrocarbonée contient des groupes substituants choisis parmi les alcools, les thiols, les éthers, les amines et des fractions acides carboxyliques.

4. Procédé selon l'une quelconque des revendications précédentes pour convertir des alcanes en C₁ à C₄ en hydrocarbures de plus haut poids moléculaire, le procédé comprenant les étapes consistant à :
(a) mettre en contact un ou plusieurs alcanes en C₁ à C₄ ;
un agent oxydant qui est HNO₃, O₂, SO₃, H₂SO₄ ou H₂SeO₄ ;
un acide résistant à l'oxydation, ayant un pKa < 3,0 ; et
un catalyseur, au moins en une quantité catalytique, comprenant un ou plusieurs des métaux Cu, Zn, Pd, Ag, Cd, In, Sn, Sb, Te, Pt, Au, Hg, Tl, Pb, Bi, Ga, Ge, As, Po, Rh, Ir, Os ou Ru ou un cation de ceux-ci ;
dans des conditions d'estérification pour produire un alkyl oxyester de l'acide en une quantité molaire plus grande que la quantité molaire du catalyseur ;
(b) convertir l'alkyl oxyester de l'acide en un produit intermédiaire alkylique ; et
(c) convertir catalytiquement le produit intermédiaire alkylique en hydrocarbures de plus haut poids moléculaire.

5. Procédé pour estérifier un ou plusieurs alcanes en C₁ à C₄, le procédé comprenant les étapes consistant à :
(a) mettre en contact lesdits un ou plusieurs alcanes en C₁ à C₄ ;
un ou plusieurs des agents oxydants HNO₃, O₂, SO₃, H₂SO₄ ou H₂SeO₄ ;
un acide résistant à l'oxydation, ayant un pKa < 3,0 ; et
un catalyseur, au moins en une quantité catalytique, comprenant un ou plusieurs des métaux Cu, Zn, Pd, Ag, Cd, In, Sn, Sb, Te, Pt, Au, Hg, Tl, Pb, Bi, Ga, Ge, As, Po, Rh, Ir, Os ou Ru ou un cation de ceux-ci ;
dans des conditions d'estérification pour produire un alkyl oxyester de l'acide en une quantité molaire plus grande que la quantité molaire du catalyseur ; et
(b) récupérer l'alkyl oxyester de l'acide.

6. Procédé selon la revendication 4 ou 5, dans lequel l'alcane comprend le méthane.

7. Procédé selon la revendication 6, dans lequel le produit intermédiaire alkylique comprend le méthanol.

8. Procédé selon la revendication 4 ou 5, dans lequel l'alcane comprend l'éthane, le propane ou le butane.

9. Procédé selon l'une quelconque des revendications précédentes, dans lequel l'oxydation est effectuée à l'anode d'une cellule électrochimique.

10. Procédé selon l'une quelconque des revendications précédentes, dans lequel l'agent oxydant est O₂.

11. Procédé selon l'une quelconque des revendications 1 à 9, dans lequel l'agent oxydant est SO₃.

12. Procédé selon l'une quelconque des revendications 1 à 9, dans lequel l'agent oxydant est H₂SO₄.

13. Procédé selon l'une quelconque des revendications précédentes, dans lequel l'acide est un acide solide.

14. Procédé selon l'une quelconque des revendications précédentes, dans lequel l'acide est HF, HCl, HBr, H₂SeO₄, HNO₃, H₂SO₄, CF₃CO₂H, CF₃SO₃H, H₃PO₄, les HPA (hétéro-polyacides), B(OH)₃, un anhydride ou sel acide de ces acides, et les mélanges de deux ou plus de ces acides ou de ces acides et anhydrides.

15. Procédé selon la revendication 14, dans lequel l'acide est H₂SO₄.

16. Procédé selon la revendication 14, dans lequel l'acide est H₄P₂O₇.

17. Procédé selon la revendication 14, dans lequel l'acide est CF₃SO₃H.

18. Procédé selon l'une quelconque des revendications précédentes, dans lequel le métal catalytique est Pd, Tl, Pt, Hg ou Au, ou un cation de ceux-ci.

19. Procédé selon la revendication 18, dans lequel le métal catalytique est Hg ou un cation de celui-ci.

20. Procédé selon l'une quelconque des revendications précédentes, dans lequel l'étape (a) se déroule en présence de l'étape de conversion de l'alkyl oxyester dans (b) de sorte que l'alkyl oxyester est simultanément converti en le produit intermédiaire alkylique sans isolement de l'alkyl oxyester.
